(19) Europäisches Patentamt
European Patent Office
Office européen des brevets

(11) Veröffentlichungsnummer: **0 315 908 B1**

(12) **EUROPÄISCHE PATENTSCHRIFT**

(45) Veröffentlichungstag der Patentschrift: **26.08.92**

(51) Int. Cl.[5]: **C07C 59/135**, C07C 51/58

(21) Anmeldenummer: **88118391.7**

(22) Anmeldetag: **04.11.88**

# (54) Verfahren zur Herstellung von perfluorierten Carbonsäurefluoriden.

(30) Priorität: **07.11.87 DE 3737920**

(43) Veröffentlichungstag der Anmeldung:
**17.05.89 Patentblatt 89/20**

(45) Bekanntmachung des Hinweises auf die Patenterteilung:
**26.08.92 Patentblatt 92/35**

(84) Benannte Vertragsstaaten:
**BE CH DE FR GB IT LI NL**

(56) Entgegenhaltungen:
**EP-A- 0 150 618**
**DE-A- 3 130 859**

**PATENT ABSTRACTS OF JAPAN, Band 10, Nr. 230 (C-365)[2286], 9. August 1986; & JP-A-61 65 841 (DAIKIN IND. LTD) 04-04-1986**

**Angew. Chem. Int. Ed. Engl. 24, S.165**

(73) Patentinhaber: **HOECHST AKTIENGESELLSCHAFT**
**Postfach 80 03 20**
**W-6230 Frankfurt am Main 80(DE)**

(72) Erfinder: **Kruse, Alfred, Dr.**
**Hornauer Strasse 199**
**W-6233 Kelkheim (Taunus)(DE)**
Erfinder: **Siegemund, Günter, Dr.**
**Frankfurter Strasse 21**
**W-6238 Hofheim am Taunus(DE)**
Erfinder: **Schwertfeger, Werner, Dr.**
**Erlenstrasse 8**
**W-6306 Langgöns(DE)**

**EP 0 315 908 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von perfluorierten Carbonsäurefluoriden der Formel

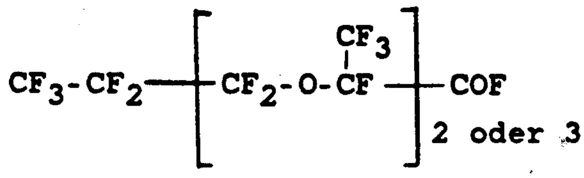

(I)

durch Oligomerisation von Hexafluorpropenoxid in Gegenwart eines Katalysators. Die beiden genannten Verbindungen sind somit das Trimer und das Tetramer des Hexafluorpropenoxids. Sie sind wertvolle Zwischenprodukte auf dem Gebiet der organischen Fluorchemie. Insbesondere werden sie eingesetzt zur Herstellung von Perfluorpolyethern, die z.B. als Inertflüssigkeiten verwendet werden,oder zur Herstellung von perfluorierten Vinylethern,die als Comonomere zur Herstellung von fluorierten Kunststoffen dienen.

Die Oligomerisierung von Hexafluorpropenoxid (HFPO) in Gegenwart verschiedener Katalysatoren ist bereits bekannt und läuft nach der folgenden Gleichung ab:

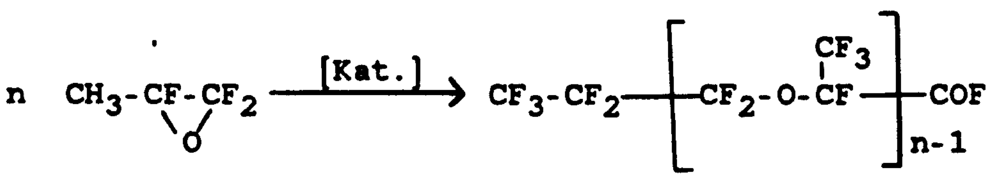


Eine zusammenfassende Darstellung dieser Reaktion einschließlich der verwendeten Katalysatoren ist in Angew. Chem. Int. Ed. Engl. 24, 161-178 (1985) (insbesondere S. 165, 166) gegeben worden. Die genannten Einsatzmöglichkeiten für das Trimer und das Tetramer werden dort auf S. 176-177 referiert.

Die selektive Dimerisierung von HFPO ist bereits bekannt (US-PS 4 081 467, US-PS 3 896 179). Bei der weiteren Oligomerisation von HFPO entstehen jedoch im allgemeinen Gemische mit hohem Molekulargewicht und/oder breitem Produktspektrum.

Nach der DE-OS 2 627 986 lassen sich das Trimer und das Tetramer der Formel (I) durch Polymerisation von HFPO unter der katalytischen Wirkung von Bisdialkylaminodifluormethanen erhalten. Nachteilig ist bei diesem Verfahren, daß sehr lange Reaktionszeiten, tiefe Temperaturen von -20 °C bis -30 °C, eine aufwendige Katalysatorherstellung, sowie der Einsatz von Hexafluorpropen/HFPO-Gemischen notwendig sind.

Es ergab sich nun die Aufgabe, für eine kommerziell verwertbare Oligomerisierung von HFPO zum Trimer sowie zum Tetramer ein leistungsstarkes, möglichst selektiv wirkendes Katalysatorsystem zu finden.

Gegenstand der Erfindung ist ein Verfahren zur Herstellung von perfluorierten Carbonsäurefluoriden der Formel

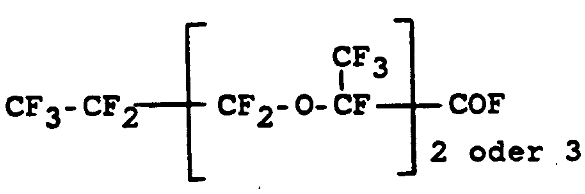

(I)

durch Oligomerisation von Hexafluorpropenoxid in Gegenwart eines Katalysators, dadurch gekennzeichnet, daß der Katalysator aus einem Alkalifluorid, einem Carbonsäuredinitril und einem Ether der Formel

$CH_3\text{-}O\text{-}(CH_2CH_2\text{-}O)_x\text{-}CH_3$ (II)

besteht, in dem x eine ganze Zahl von 2 bis 6 bedeutet.

Ein derartiges Katalysatorsystem wurde bereits bei der selektiven Addition von HFPO an perfluorierte Carbonsäurefluoride eingesetzt. (EP-A-0 070 635, DE-OS 31 30 859, EP-A-0 150 618). Der Vorteil dieses

Katalysatorsystems soll dort die selektive Addition von HFPO an das eingesetzte Carbonsäurefluorid bei gleichzeitig geringem Anfall von unerwünschten Oligomerisationsprodukten des HFPO sein. Es war daher überraschend, daß gerade dieses Katalysatorsystem in der Lage ist, die gezielte Oligomerisierung von HFPO zum Trimer oder zum Tetramer sowie hohe Umsatzraten an HFPO zu bewirken.

Nach Tab.2 der oben erwähnten Literaturstelle Angew. Chem. Int. Ed. Engl. 24, S. 165 werden in Gegenwart von Tetraglyme nur 42% Tetramer und Trimer gebildet und nach Tab.3 in Gegenwart von Nitril nur 63.4%. Überraschenderweise ist beim erfindungsgemäßen Verfahren in Gegenwart von sowohl Tetraglyme als auch Dinitril die Selektivität über 70%.

Bei dem erfindungsgemäßen Verfahren erfolgt die Oligomerisierung von HFPO mit einem Katalysatorsystem, das aus einem Gemisch aus einem Alkalifluorid, einem Carbonsäuredinitril und einem Polyethylenglykoldimethylether besteht. Als Alkalifluoride sind für das erfindungsgemäße Katalysatorsystem vor allem Kaliumfluorid, Natriumfluorid und Caesiumfluorid geeignet, insbesondere Kaliumfluorid. Als Carbonsäuredinitrile werden vorzugsweise die Dinitrile von gesattigten aliphatischen Dicarbonsäuren mit 5 bis 8 Kohlenstoffatomen, vorzugsweise Adipinsäuredinitril verwendet. Bevorzugte Polyethylenglykoldimethylether sind der Dimethylether des Tri-, Tetra- oder Pentaethylenglykols, insbesondere Tetraethylenglykoldimethylether.

Die drei Komponenten des Katalysatorsystems werden vorzugsweise im Mengenverhältnis von (2-30) Gew.-% Alkalifluorid : (50-95) Gew.-% Dinitril : (2-50) Gew.-% Polyether, bezogen auf das gesamte Katalysatorsystem, eingesetzt.

Man erhält ein Gemisch aus Trimer und Tetramer, das noch Anteile von Dimer und Pentamer enthält. Dabei führt eine größere Konzentration an Polyether im Katalysatorsystem zur Erhöhung des mittleren Oligomerisationsgrades und der Reaktionsgeschwindigkeit. Auf diese Weise lassen sich durch Reaktion in Gegenwart geringerer Konzentrationen an Polyether das Trimer oder durch Reaktion in Gegenwart größerer Konzentrationen an Polyether das Tetramer gezielt als Hauptkomponenten erhalten. Gleichzeitig wird mit diesem Katalysatorsystem eine enge Molekulargewichtsverteilung erreicht. Erhöhung des relativen Gewichtsanteils an Alkalifluorid im Katalysatorgemisch bei konstantem Verhältnis der beiden anderen Katalysatorkomponenten führt ebenfalls zur Erhöhung des mittleren Oligomerisationsgrades.

Die relative Zusammensetzung des Oligomerengemisches läßt sich weiterhin auch durch Wahl der Reaktionstemperatur steuern. Erhöhung der Reaktionstemperatur führt zur Erniedrigung des Oligomerisationsgrades, Erniedrigung der Reaktionstemperatur bewirkt das Umgekehrte.

Ein weiterer wesentlicher Vorteil des erfindungsgemäßen Verfahrens neben der Selektivität ist die einfache Aufarbeitung des Reaktionsgemisches durch Phasentrennung, da Reaktionsprodukt und Katalysatormischung kaum ineinander löslich sind und ein zweiphasiges Gemisch bilden. Die Katalysatorlösung kann auf diese Weise mehrfach wiederverwendet werden; damit ist auch die Möglichkeit einer kontinuierlichen Reaktionsführung gegeben. Ein besonderer Vorteil ist die Verwendung von einfachen und preiswerten Katalysatormaterialien.

Das Katalysatorsystem kann als solches eingesetzt, oder auch mit einem inerten Lösungsmittel verdünnt werden, das ein geringes Lösungsvermögen für die beiden oligomeren Carbonsäurefluoride aufweist, wie z.B. aliphatische oder aromatische Kohlenwasserstoffe.

Es ist zweckmäßig, das Alkalifluorid vor dem Einsatz scharf zu trocknen und fein zu pulverisieren. Das Carbonsäuredinitril und der Ether können in handelsüblicher Reinheit direkt eingesetzt werden. Der Einfluß einer etwaigen Restfeuchte des Katalysatorsystems auf die mittlere Oligomerenverteilung kann durch Variation der Reaktionstemperatur oder der Zusammensetzung des Katalysatorgemisches im gewünschten Sinne korrigiert werden.

Zur Durchführung des erfindungsgemäßen Verfahrens legt man in einem Gefäß, das z.B. aus Glas oder rostfreiem Edelstahl bestehen kann und das mit einem effektiven Rührer ausgestattet ist, das Katalysatorgemisch vor und dosiert das HFPO-Gas zu. Es ist von Vorteil, während der gesamten Reaktion kräftig zu rühren. Die Umsetzung von HFPO verläuft exotherm; die Reaktionstemperatur kann zwischen -20 °C bis 100 °C liegen, bevorzugt wird bei Temperaturen zwischen 0 °C und 80 °C gearbeitet.

Die Reaktion kann bei Normaldruck durchgeführt werden. Für einen zügigen Umsatz ist jedoch erhöhter Druck günstig; im allgemeinen wird man höchstens beim Eigendruck von HFPO arbeiten. Der Druck im Reaktionsgefäß kann durch Einstellung der Nachschubgeschwindigkeit an gasförmigem HFPO kontrolliert werden.

Das Gemisch der oligomeren Carbonsäurefluoride der Formel (I) scheidet sich als untere Phase ab und kann durch ein Bodenventil des Reaktionsgefäßes abgelassen werden.

Das Katalysatorsystem kann daher einfach abgetrennt und mehrfach verwendet werden. Dabei schadet es nicht, wenn neben dem Katalysatorgemisch auch eine restliche Menge an Produkt für den nächsten Ansatz im Reaktionsgefäß verbleibt.

Da Alkalifluorid und Ether bei mehrfacher Verwendung des Katalysatorgemisches teilweise mit dem Produkt ausgetragen werden, was zu einer Verschiebung der Produktzusammensetzung führen würde, kann es notwendig sein, eine oder beide Komponenten vor einem weiteren Versuch zu ergänzen.

Eine kontinuierliche Durchführung der Reaktion ist möglich, wenn über eine Beruhigungszone ständig die untere Produktphase aus dem Reaktionsgefäß ausgeschleust und im gleichen Maß HFPO zudosiert wird.

Die weitere Aufarbeitung erfolgt im allgemeinen durch destillative Trennung der oligomeren Säurefluoride, die auf diese Weise in Reinheiten über 99 Gew.-% erhalten werden können.

**Beispiel 1**

In einem mit einem Rührer ausgestatteten 5 l-Sathlautoklaven wurden 30 g Kaliumfluorid, 500 ml Adipinsäuredinitril und 100 ml Tetraethylenglykoldimethylether gegeben und 30 min. gerührt. Anschließend wurde unter gutem Rühren HFPO unter Druck zugegeben, wobei eine exotherme Reaktion einsetzte. Die Innentemperatur des Reaktors wurde durch Außenkühlung bei 35-40 °C gehalten, der Druck wurde durch Regelung des Zustroms an gasförmigem HFPO auf 3,5 bar Überdruck im Reaktionsgefäß eingestellt. Insgesamt wurden innerhalb von 2 1/2 Stunden 5 kg HFPO eindosiert. Der Ansatz wurde anschließend bis zur Abnahme des Überdrucks (ca. 3 Stunden) nachgerührt. Das Reaktionsgemisch trennte sich rasch in zwei Phasen. Die untere Phase (4,90 kg) mit dem Reaktionsprodukt wurde durch ein Bodenventil abgelassen (die obere Phase ist die Katalysatormischung). Das Reaktionsprodukt wies (gemäß Gaschromatographie einer mit Methanol veresterten Probe) folgende Zusammensetzung auf:

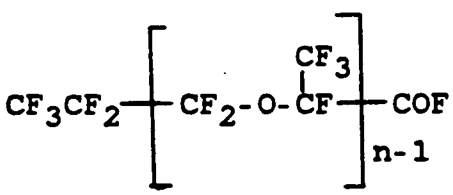

| | Gew.-% | Kp. |
|---|---|---|
| n = 2 | 21,5 | 56 - 57 °C |
| n = 3 | 61,1 | 114 - 116 °C |
| n = 4 | 16,3 | 162 - 163 °C |
| n = 5 | 0,8 | 201 - 203 °C |

**Beispiel 2**

In der gleichen Apparatur wie in Beispiel 1 wurde unter sonst gleichen Bedingungen die Oligomerisierung bei einer Reaktionstemperatur von 20 - 25 °C durchgeführt. Es wurden innerhalb von 2 Stunden 5 kg HFPO eindosiert. Der Ansatz wurde bis zur Abnahme des Überdrucks (ca. 2 Stunden) nachgerührt. Die untere Phase (4,93 kg) mit dem Reaktionsprodukt wurde abgelassen. Das Reaktionsprodukt wies (gemäß Gaschromatographie einer mit Methanol veresterten Probe) folgende Zusammensetzung auf:

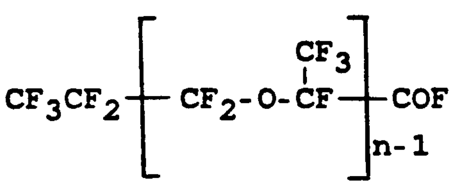

| | Gew.-% |
|---|---|
| n = 2 | 15,2 |
| n = 3 | 56,8 |
| n = 4 | 25,8 |
| n = 5 | 2,1 |

**Beispiel 3**

In der gleichen Apparatur wie in Beispiel 2 wurde ein Katalysatorgemisch aus 50 g Kaliumfluorid, 400 ml Adipinsäuredinitril und 200 ml Tetraethylenglykoldimethylether vorgelegt. Innerhalb von 2 Stunden wurden 5 kg HFPO eindosiert, wobei die Innentemperatur durch Kühlung des Reaktionsgefäßes bei 20-30 °C gehalten wurden. Der Druck wurde bei 1 bis 2 bar Überdruck gehalten. Der Ansatz wurde bis zur Abnahme des Überdrucks (ca. 1/2 Stunde) nachgerührt. Die Produktphase (4,94 kg) wurde abgetrennt. Das Reaktionsprodukt wies (gemäß einer mit Methanol veresterten Probe) folgende Zusammensetzung auf:

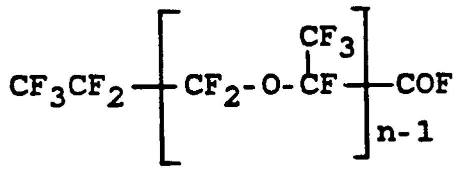

| | Gew.-% |
|---|---|
| n = 2 | 3,1 |
| n = 3 | 26,2 |
| n = 4 | 47,7 |
| n = 5 | 19,7 |
| n = 6 | 3,1 |

**Beispiel 4**

In einen mit einem Rührer ausgestatteten 2 l-Autoklav wurden 50 g Kaliumfluorid, 300 ml Adipinsäuredinitril und 10 ml Tetraethylenglykoldimethylether gegeben und 30 Minuten gerührt. Unter gutem Rühren wurde HFPO eindosiert. Die Innentemperatur des Reaktors wurde durch Außenkühlung bei 35 bis 40 °C gehalten, der Druck im Reaktionsgefäß wurde auf 3,5 bar Überdruck eingestellt. Innerhalb von 2 Stunden wurde 1,5 kg HFPO eindosiert. Der Ansatz wurde 1/2 Stunde nachgerührt, dann wurde 1 kg der unteren Phase mit dem Reaktionsprodukt durch ein Bodenventil abgelassen. Unter den vorstehenden Reaktionsbedingungen wurde dann wieder 1 kg HFPO in das Reaktionsgefäß eindosiert. Dieser Vorgang wurde mehrfach wiederholt, wobei nach jeder zweiten Charge 10 ml Tetraethylenglykoldimethylether nachgefüllt wurden. Die Produktphase der ersten Charge und die Produktphase nach 14-facher Wiederholung des Zyklus wiesen folgende Zusammensetzung auf (jeweils in Gew.-%):

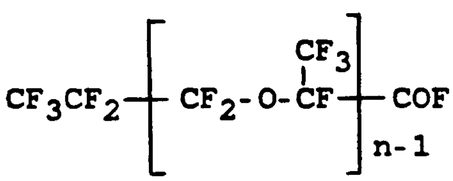

| | 1. Charge | 14. Charge |
|---|---|---|
| n = 2 | 18,9 | 23,2 |
| n = 3 | 60,6 | 59,2 |
| n = 4 | 19,6 | 16,8 |
| n = 5 | 0,9 | 0,7 |

**Patentansprüche**

1. Verfahren zur Herstellung von perfluorierten Carbonsäurefluoriden der Formel

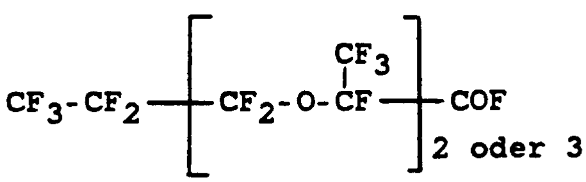

(I)

durch Oligomerisation von Hexafluorpropenoxid in Gegenwart eines Katalysators, dadurch gekennzeichnet, daß der Katalysator aus einem Alkalifluorid, einem Carbonsäuredinitril und einem Ether der Formel

$CH_3$-O-($CH_2CH_2$-O)$_x$-$CH_3$ (II)

besteht, in dem x eine ganze Zahl von 2 bis 6 bedeutet.

2. Verfahren nach Anspruch 1, dadurch gekennzeichnet, daß als Alkalifluorid Kaliumfluorid verwendet wird.

3. Verfahren nach Anspruch 1 oder 2, dadurch gekennzeichnet, daß als Carbonsäuredinitril ein Dinitril einer gesättigten aliphatischen Dicarbonsäure mit 5 bis 8 Kohlenstoffatomen verwendet wird.

4. Verfahren nach Anspruch 1 oder 2, dadurch gekennzeichnet, daß als Carbonsäuredinitril Adipinsäuredinitril verwendet wird.

5. Verfahren nach mindestens einem der Anspruch 1 bis 4, dadurch gekennzeichnet, daß als Ether der Formel (II) den Dimethylether des Tri-, Tetra- oder Pentaethylenglykols verwendet wird.

6. Verfahren nach mindestens einem der Ansprüche 1 bis 4, dadurch gekennzeichnet, daß man als Ether der Formel (II) Tetraethylenglykoldimethylether verwendet.

7. Verfahren nach mindestens einem der Ansprüche 1 bis 6, dadurch gekennzeichnet, daß die Komponenten des Katalysatorsystems in einer Menge von 2-30 Gew.-% Alkalifluorid, 50-95 Gew.-% Carbonsäuredinitril und 2-50 Gew.-% Ether verwendet werden, bezogen auf das gesamte Katalysatorsystem.

8. Verfahren nach mindestens einem der Ansprüche 1 bis 7, dadurch gekennzeichnet, daß die Umsetzung bei einer Temperatur von -20 °C bis +100 °C durchgeführt wird.

## Claims

1. A process for the preparation of perfluorinated carbonyl fluorides of the formula

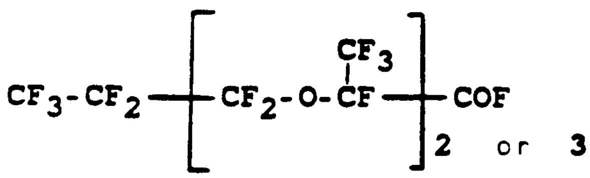

(I)

by oligomerisation of hexafluoropropene oxide in the presence of a catalyst, wherein the catalyst comprises an alkali metal fluoride, a carboxylic acid dinitrile and an ether of the formula

$CH_3\text{-}O\text{-}(CH_2CH_2\text{-}O)_x\text{-}CH_3$ (II)

in which x denotes an integer from 2 to 6.

2. The process as claimed in claim 1, wherein potassium fluoride is used as alkali metal fluoride.

3. The process as claimed in claim 1 or 2, wherein a dinitrile of a saturated aliphatic dicarboxylic acid having 5 to 8 carbon atoms is used as carboxylic acid dinitrile.

4. The process as claimed in claim 1 or 2, wherein adiponitrile is used as carboxylic acid dinitrile.

5. The process as claimed in at least one of claims 1 to 4, wherein the dimethyl ether of tri-, tetra- or pentaethylene glycol is used as ether of the formula (II).

6. The process as claimed in at least one of claims 1 to 4, wherein tetraethylene glycol dimethyl ether is used as ether of the formula (II).

7. The process as claimed in at least one of claims 1 to 6, wherein the components of the catalyst system are used in an amount of 2-30% by weight of alkali metal fluoride, 50-95% by weight of carboxylic acid dinitrile and 2-50% by weight of ether, relative to the total catalyst system.

8. The process as claimed in at least one of claims 1 to 7, wherein the reaction is carried out at a temperature of -20 °C to +100 °C.

## Revendications

1. Procédé pour préparer des fluorures d'acides carboxyliques perfluorés de formule I :

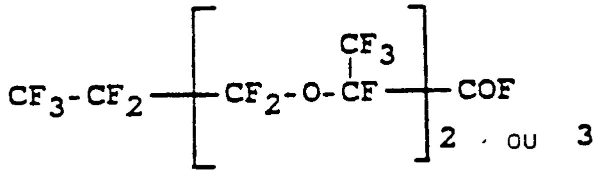 (I)

par oligomérisation de l'oxyde d'hexafluoro-propène en présence d'un catalyseur, procédé caractérisé en ce que le catalyseur est constitué d'un fluorure de métal alcalin, d'un bis-carbonitrile et d'un éther répondant à la formule II :

$CH_3$-O-($CH_2CH_2$-O)$_x$-$CH_3$ (II)

dans laquelle x désigne un nombre entier de 2 à 6.

**2.** Procédé selon la revendication 1 caractérisé en ce qu'on utilise du fluorure de potassium comme fluorure de métal alcalin.

**3.** Procédé selon l'une des revendications 1 et 2, caractérisé en ce qu'on utilise, comme bis-carbonitrile, un dinitrile d'un acide dicarboxylique aliphatique saturé contenant de 5 à 8 atomes de carbone.

**4.** Procédé selon l'une des revendications 1 et 2, caractérisé en ce qu'on utilise l'adiponitrile comme bis-carbonitrile.

**5.** Procédé selon au moins une des revendications 1 à 4, caractérisé en ce qu'on utilise, comme éther de formule II, l'éther diméthylique du diéthylène-glycol, du tétraéthylène-glycol ou du pentaéthylène-glycol.

**6.** Procédé selon au moins une des revendications 1 à 4, caractérisé en ce qu'on utilise, comme éther de formule II, l'éther diméthylique du tétraéthylène-glycol.

**7.** Procédé selon au moins une des revendications 1 à 6, caractérisé en ce que les composantes du système catalytique sont utilisées en une quantité de 2 à 30% en poids pour le fluorure de métal alcalin, de 50 à 95% en poids pour le bis-carbonitrile et de 2 à 50% en poids pour l'éther, par rapport au système catalytique total.

**8.** Procédé selon au moins une des revendications 1 à 7, caractérisé en ce que la réaction est effectuée à une température comprise entre -20 et +100 ° C.